# EUROPEAN PATENT SPECIFICATION

(11) **EP 1 749 551 B1**
(45) Date of publication and mention of the grant of the patent: **19.03.2008**
(21) Application number: 06011025.1
(22) Date of filing: 29.05.2006
(51) Int. Cl.: A61N 5/10, A61H 33/00

(54) **Method for the preparation of water having radioactivity for use in bathing**
Verfahren zur Aufbereitung von Radioaktivität enthaltendem Badewasser
Procédé de traitement des eaux de baignade contenant de radioactivité

(30) Priority: 01.08.2005 JP 2005223419
(43) Date of publication of application: 07.02.2007
(73) Proprietor: HEALTHY PEOPLE CO., LTD., Chuo-ku Tokyo (JP)
(72) Inventor: Tamura, Hikaru, Chigasaki-shi Kanagawa (JP); Yamamoto, Koji, Toshima-ku, Tokyo (JP)
(74) Representative: Beetz & Partner

(56) References cited:
- JP-A- 55 007 223
- JP-A- 2005 124 898
- US-A- 5 457 323

## Description

The present invention relates to a method for the preparation of water having radioactivity for use in bathing containing a radioactive isotope or isotopes of a short half-life which are not accumulated in the body and hence are free of any physiologically bad influence.

From the past, it is well known that radioactive hot or warm bathing as in natural radium spas as scattered all over the country is extremely efficacious against various disorders.

However, these natural radium spas irrespective of whether they are hot or cold gush out only in the limited places so that users cannot use these spas at a desired time and a desired place. Accordingly, there have been proposed hitherto a variety of trials for artificially preparing water having radioactivity for use in bathing, which has various radioactive substances and/or radioactive decay products dissolved therein to exhibit the equivalent efficacy to natural radium spas. For example, below are such trials for preparing water having radioactivity for use in bathing.

In JP55-42613A, there is disclosed a method for extracting the decay products from radioactive substances by bringing the radioactive substances into contact with an aqueous solution of an organic acid, wherein the temperature of the extraction liquid, the concentration of the organic acid, pH value and radioactive dose are adjusted to be within ranges suitable for bathing.

In JP59-171561A, there is disclosed a method for preparing an aqueous solution possessing radioactivity by contacting a material containing radioactive isotopes with an aqueous solution of an organic acid to extract radioactive decay products in the material containing radioactive isotopes with the aqueous solution by the aid of a fluidization-circulation-type contacting extractor having a cylindrical container provided with a filter in the lower part thereof, a feeding port for a liquid in the upper part thereof, an inlet port for a liquid on the bottom thereof, and a circulation pipeline connecting the feeding port and the inlet port via a pump, which method comprises putting a particulate material containing radioactive isotopes on the filter in the apparatus, feeding the aqueous solution of the organic acid to the apparatus so that the liquid level of the solution may be located in a position higher than the feeding port of the liquid, and then circulating the aqueous solution of the organic acid by way of the pump and the circulation pipeline of the apparatus so as to form a fluidized bed of the particulate material containing radioactive isotopes.

In JP2-32300A, there is disclosed a method for preparing water containing radioactive decay products in an extracting tank arranged with a stirring tank, which method comprises placing granules of an ore containing radioactive material such as thoron or radon, feeding a hot acidic extracting solution to the extracting tank to such a degree that the stirring tank is at least sunk, supplying a gas under pressure to the tank so that a jet stream may be formed in the stirring tank to agitate the granules of the ore thereby permitting collapse of ascending bubbles evolved from the gas to impart ultrasonic vibration to the ore, and accelerating decay of the radioactive materials contained in the ore to enable extraction of the radioactive decay products in the form of a gas while dissolving a part of the gas in the hot extracting solution.

In JP2001-37844A and JP2001-70464A, there is disclosed an apparatus provided with an extracting tank filled with an acidic aqueous solution and being capable of contacting an ore powder containing radioactive materials with the acidic aqueous solution to extract radioactive decay products and a stirring tank having a wall a part of which is constructed by a filter and filled with the ore powder.

In JP2005-124898A, there is disclosed a method for preparing water for use in artificial hot bathing which comprising the steps of feeding a given amount of an extracting agent to an extracting tank, supplying fresh water to the extracting tank until the liquid level of the water reaches to a predetermined point, starting a circulation pump and at the same time sending electricity to a heater and a temperature controlling device of the extracting tank and further to a heater and a temperature controlling device of a storage tank, releasing a solenoid valve (A1) mounted to a pipeline between the storage tank and the circulation pump and a solenoid valve (A) mounted to a pipeline between the circulation pump and the extracting tank, preparing water for use in an artificial hot bathing by sending water from the storage tank to the extracting tank for a given period of time by the aid of the circulating pump in such manner that water in the extracting tank may go back over a bulkhead between the extracting tank and the storage tank to the storage tank, cutting power supply to the heater and the temperature controlling device of the extracting tank and to the heater and the temperature controlling device of the storage tank and stopping the action of the circulation pump to shut down the solenoid valve (A1) and the solenoid valve (A), filling a bath tank with fresh water or warm water, starting the circulation pump and at the same time sending electricity to the heater and the temperature controlling device of the storage tank, releasing a solenoid valve (B1) mounted to a pipeline between the bath tank and the circulation pump and a solenoid valve (B) mounted to the pipeline between the circulation pump and the storage tank to send the water or warm water from the bath tank to the storage tank for a given period of time by way of the circulation pump whereby uniform water for use in artificial hot bathing is prepared by circulating the water in such manner that the water for use in artificial hot bathing in the bath tank may go back over a given water level of the storage tank to the bath tank through a pipeline provided between the storage tank and the bath tank, and then cutting power supply to the heater and the temperature controlling device of the storage tank while stopping the action of the circulation pump to shutting down the solenoid valve (B1) and the solenoid valve (B).

In these methods are chiefly used easily commercially available minerals containing radioactive substances such as monazite (a phosphate mineral of Ce, La, Nd, and Th) which are of mild effect and are harmless to human body and xenotime (a phosphate mineral of Y, Ce, U, and Th).

Regarding the therapeutic effect of radioactive spa remedy, it has been considered heretofore for a long period of time that α-rays emitted by radioactive substances penetrate human body to stimulate individual living tissue cells to excite their physiological function. It has therefore been recommended to employ a radioactive mineral enriched in the content of radon-series isotopes having a long half-life and capable of emitting α-rays for a long period time as radioactive source.

It has been made clear, however, that the existence of Ion Channel was confirmed by the study of the eminent scientists of Dr. Elwin Neher and Dr. Bert Sakman, both winning Nobel Prize, and the function mechanism of the ion channel was also resolved. As the result, the theory established hitherto in the field of the functional physiology was drastically changed.

It is noteworthy that the ion channel is an intracellular and extracellular physiological tunnel existing in cellular membrane of cells and permits a function of intrusion of cations such as metal ions into cells on demand due to a physiological action as filter for anions. An inner diameter of the ion channel is approximately 5 x 10⁻⁸ cm and enables to intrusion of divalent cations of radon and thorium in addition to electrolytic metal ions such as sodium ion, potassium ion, and calcium ion into cells. As the result, it is found that the theory of therapeutic efficacy trusted hitherto based on the intrusion of α-rays only was denied, but the direct effect of radon ion and/or thorium ion intruded into cells through the ion channel are now understood. Accordingly, it is now contrarily anticipated that the use of a radioactive substance having a long half-life gives physiologically harmful effect since repeated hot bathing of water containing such radioactive substances permits build-up of such radioactive substances in cells.

In view of the foregoing situations, it is an object of the present invention to eliminate radon-series isotopes having a long half-life and a fear of being accumulated in the living body to exhibit physiologically harmful effect from a natural radioactive ore containing both of thoron-series isotopes and radon-series isotopes.

It is another object of the present invention to provide water having radioactivity for use in bathing which is physiologically safe even if it is repeatedly used for bathing and does not permit build-up of harmful radioactive isotopes of a long half-life in the living body.

As a result of extensive research made to develop a new type substitute for natural radioactive hot spa devoid of physiologically harmful action, it has now been found that a natural radioactive ore containing both of thoron-series isotopes and radon-series isotopes can be treated for selectively eliminating only the radon-series isotopes having a long half-life and the ore thus treated can be contacted with an aqueous solution of an organic acid to extract radioactive decay products contained in the ore. The resultant aqueous solution exhibits radioactivity coming from the treated natural radioactive ore free or a low content of radon-series isotopes and can be used for a substitute of the natural radioactive spas, thus achieving the object of the invention. The present invention has now been accomplished on the basis of the above finding.

In accordance with one embodiment of the present invention, there is provided a method for the preparation of water having radioactivity for use in bathing enriched in the content of thoron-series isotopes which comprises the steps of (A) subjecting a natural radioactive ore containing both of thoron-series isotopes and radon-series isotopes to a separation treatment for selectively eliminating the radon-series isotopes only thereby decreasing the content of the radon-series isotopes and (B) bringing the natural radioactive ore thus treated into contact with an aqueous solution of an organic acid to extract radioactive decay products in the natural radioactive ore with the aqueous solution.

In accordance with one aspect of the embodiment, there is provided a method for the preparation of water having radioactivity for use in bathing, wherein the natural radioactive ore used in the step (A) is a heavy sand collected from a pegmatite-based sand deposit and the step (A) is conducted by separation of thorium-series monazite from uranium-series xenotime through mineral dressing in such manner that the amount of the xenotime is reduced to decrease the content of the radon-series isotopes.

In accordance with another aspect of the embodiment, there is provided a method for preparation of water having radioactivity for use in bathing, wherein the concentration of the aqueous solution used in the step (B) is within the range from 0.01 to 50% by weight.

In accordance with still another aspect of the embodiment, there is provided a method for the preparation of water having radioactivity for use in bathing, wherein the organic acid is citric acid.

In accordance with another embodiment of the present invention, there is provided a method for the preparation of water having radioactivity for use in bathing enriched in the content of thoron-series isotopes by the aid of a fluidization-circulation-type contacting extractor having a cylindrical container provided with a filter in the lower part thereof, an outlet port for a liquid in the upper part thereof, a circulation inlet port for a liquid at the bottom thereof, and a circulation pipeline connecting the outlet port and the circulation inlet port via a pump, which method comprises the steps of (a) putting a particulate material containing radioactive isotopes on the filter, (b) introducing an aqueous solution of an organic acid into the container so that the liquid level of the solution is higher than the outlet port thereof for the liquid, and (c) circulating the aqueous solution by way of the pump and the circulation pipeline so as to form a fluidized bed of the particulate material containing radioactive isotopes, wherein a natural radioactive ore containing thoron-series isotopes and radon-series isotopes is subjected to a separation treatment for selectively eliminating the radon-series isotopes only thereby decreasing the content of the radon-series isotopes and the resultant product is employed as the aforementioned particulate material containing radioactive isotopes.

### In the drawings:

Fig. 1 is a drawing showing a systematic explanation on the thoron-series decay and half-life;
Fig. 2 is a drawing showing a systematic explanation on the radon-series decay and half-life;
Fig. 3 is a brief cross-sectional drawing showing a preferable extraction apparatus suitable to be used for the method of the present invention; and
Fig. 4 is a flow chart showing an example for the preparation of water having radioactivity for use in bathing in accordance with the method of the present invention.

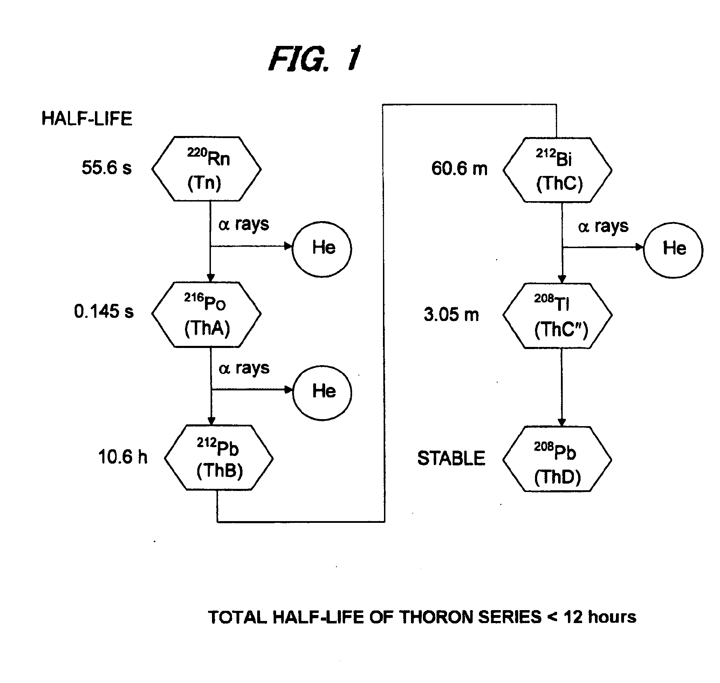

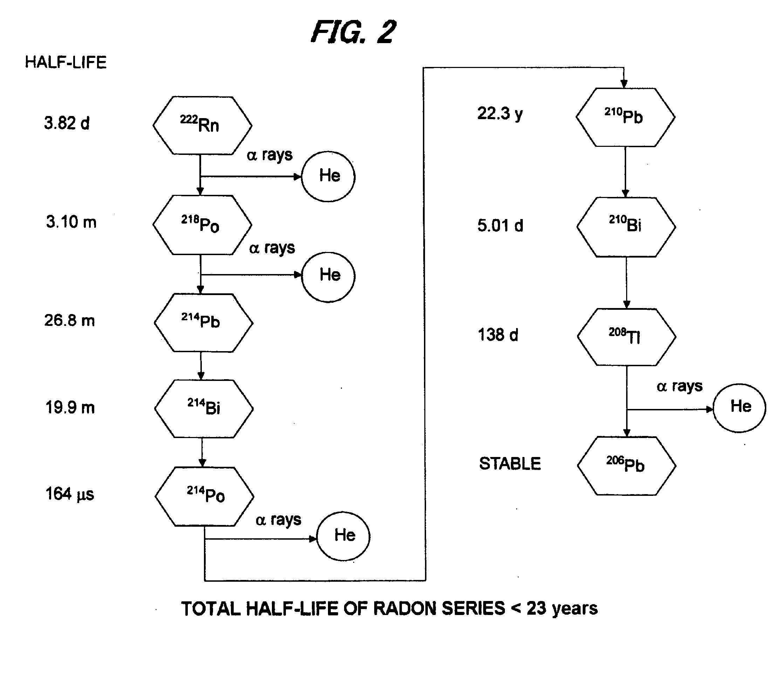

Figs. 1 and 2 show the half-life of decay in case of thorium (thoron) series elements (isotopes) and the half-life of decay in case of uranium (radon) series elements (isotopes), respectively. As is evident from comparison of Figs. 1 and 2, in the thoron-series decay, the total time of the half-lives of the thoron-series nuclides until they are stabilized does not reach 12 hours, while, in the radon-series decay, that of the radon-series nuclides until they are stabilized is about 23 years.

In the present invention, the term "thoron-series isotopes" referred to hereinafter means generic names of radioactive nuclides produced by decay of the thoron-series radioactive nuclides. Likewise, the term "radon-series isotopes" referred to hereinafter means generic names of radioactive nuclides produced by decay of the radon-series radioactive nuclides.

It follows therefore that repeated bathing in a radioactive spa abounding in the radon-series isotopes gradually causes build-up of the radon-series isotopes having a long half-life in the living body, thus giving physiologically bad influence.

According to the present invention, radon-series isotopes having a long half-life can be removed from natural radioactive ores containing throne-series isotopes and radon-series isotopes which are hitherto utilized as radioactive sources for radioactive spas so that bad influence of accumulated radioactivity on the living body can be reduced and safe water having radioactivity for use in cold or hot bathing can be offered.

The term "natural radioactive ore" referred to herein means a naturally produced ore or ores containing either or both of the thoron-series radioactive isotopes and the radon-series radioactive isotopes. As the natural radioactive ore is sometimes produced in the form of a mixture of similar ores or contaminants, it is to be construed that such mixture is also included in the category of the natural radioactive ore so far as the mixture contains the natural radioactive ore as predominant ingredient, irrespective of whether the ore may be incorporated with a purposely added radioactive substance or not. In the method of the present invention for preparing water having radioactivity for use in bathing, the natural radioactive ore can be regarded as a radioactive starting material for the method.

Accordingly, any desirable radioactive ore heretofore employed for the preparation of artificial radioactive hot spas can be used as such for the method of the present invention, and there is no limitation in the sort of natural radioactive materials. Illustrative of the natural radioactive ore usable in the present invention are, for example, ores containing uranium or radium such as uraninite, pitchblende, autunite, and planerite; and ores containing thorium such as thorite, thorogummite, and torianite. Taking easiness in availability and mildness in action into consideration, ores containing thorium such as monazite and xenotime extracted from pegmatite-based sandy ore are preferable.

The aforementioned monazite and xenotime are somewhat different in their nature and chemical composition according to their source. For example, monazite produced in Malaysia has an apparent specific gravity of 5.27 (g/cm³) and a chemical composition of 0.24 wt% of U₃O₈, 5.90 wt% of ThO₂, 59.65 wt% of total rare earth metal oxides (among which CeO₂ is 28.33 wt%) and 25.70 wt% of P₂O₅, while xenotime produced in Malaysia has an apparent specific gravity of 4.66 (g/cm³) and a chemical composition of 0.81 wt% of U₃O₈, 0.83 wt% of ThO₂, 54.05 wt% of total rare earth metal oxides (among which CeO₂ is 1.99 wt%) and 26.22 wt% of P₂O₅.

In the step (A) of the method of the present invention, it is necessary to perform a treatment for eliminating the radon-series isotopes as far as possible from the natural radioactive ore.

The content of the radon-series isotope in the natural radioactive ore is different according to the kind and source. When any kind of ores are used in the invention, the content of the radon-series isotopes is decreased as far as possible, preferably to such degree that the content of the radon-series isotopes may be less than one second or, desirably, one fifth relative of the content of thoron-series isotopes.

The treatment for eliminating the radon-series isotopes from the ore may be carried out by a specific selective extraction method, but it is advantageous to use an ore-dressing treatment wherein an ore abounding in the radon-series isotopes such as xenotime is separated from an ore abounding in the thoron-series isotopes such as monazite and the former is removed.

In case of using, for example, heavy sand collected from a pegmatite-based sand deposit as a starting material, it is subjected to a preliminary treatment wherein a rare earth ore is separated from usual phosphate ores to separate a rare earth ore. The rare earth ore is then subjected to a high gauss magnetic separation treatment followed by a high tension electrostatic separation treatment to concentrate and separate monazite and xenotime, respectively.

According to the high gauss magnetic separation treatment, an ore is subjected to a magnetic separation wherein the magnetic flux density is gradually increased within the range of 0.1-5 T to perform concentration of a magnetic ore for fractionation. In case the heavy sand collected from a pegmatite-based sand deposit, for example, is subjected to the high gauss magnetic separation treatment, iron sand is removed at 0.2-0.5 T, ilmenite is removed at 0.5-1.0 T, and finally rutile, tantalite and struverite are removed at 1.0-1.5 T.

Thus, non-magnetic ores, for example, phosphate ores, silica and zircon in an amount of about 5% by weight of the heavy sand before ore-dressing are thus retained.

The non-magnetic minerals thus retained are then subjected to the high tension electrostatic separation treatment wherein silica is removed under a lower voltage condition and minerals other than zircon and phosphate minerals are removed by gradual increase in voltage, and finally xenotime is separated from monazite by way of magnetic separation at 25,000 G (2.5 T) or higher.

Concentration and separation is continued until the content of xenotime to monazite becomes 50% by weight or less or, preferably, 20% by weight or less or, more preferably, 10% by weight or less. In this dressing process, a chemicals-insoluble and perfect non-magnetic mineral, zircon, may be co-existent. In case the content of xenotime is more than 50% by weight, safety to build-up of radioactivity is not sufficiently warranted.

In this way, the natural radioactive ore wherein the content of the radon-series isotopes is decreased is then contacted with an aqueous solution of an organic acid to dissolve radioactive substances and/or decay products contained in the ore by extraction. Illustrative of the organic acid are, for example, oxalic acid, acetic acid, citric acid, succinic acid and tartaric acid. Above all, preferable is citric acid which is colorless and odorless, harmless to human body and is easily available. The organic acid may be used singly or in the form of a mixture of two kinds or more.

The concentration of the organic acid in an aqueous solution thereof for use in extraction can be selected in the range of 0.01-50% by weight. For use in bathing, however, the concentration is preferably diluted to the range of 0.01-0.5% by weight.

The extraction treatment with the aqueous solution is carried out, for example, by crushing the natural radioactive ore reduced in the content of the radon-series isotopes, sieving the crushed material to exclude very fine particles which pass through 150 mesh screen, adding at least 2 volumetric times of an aqueous solution of an organic acid to the material, and if necessary, by applying agitation or shaking to the solution. This extraction treatment may be carried out at room temperature but is preferably carried out under heating at 50-100 °C for acceleration of extraction. The time needed for the extraction is in the range from about 30 minutes to 10 hours.

Preferable radioactive elements and their decay products contained in the extracted solution as the water for use in bathing obtained by the inventive method include the thoron-series nuclides such as ²²⁰Rn (Tn), ²¹⁶Po, ²¹²Pb, ²¹²Bi, ²⁰⁸Tl, and ²⁰⁸Pb.

As the upper limit of radioactivity defined by the related legal regulation is 370 Bq/g, radioactivity of the solution obtained according to the method of the invention has to be adjusted to 370 Bq/g or less if the solution is utilized as water for use in bathing.

Below is an explanation on one example of the apparatus for preparing the water having radioactivity for use in bathing.

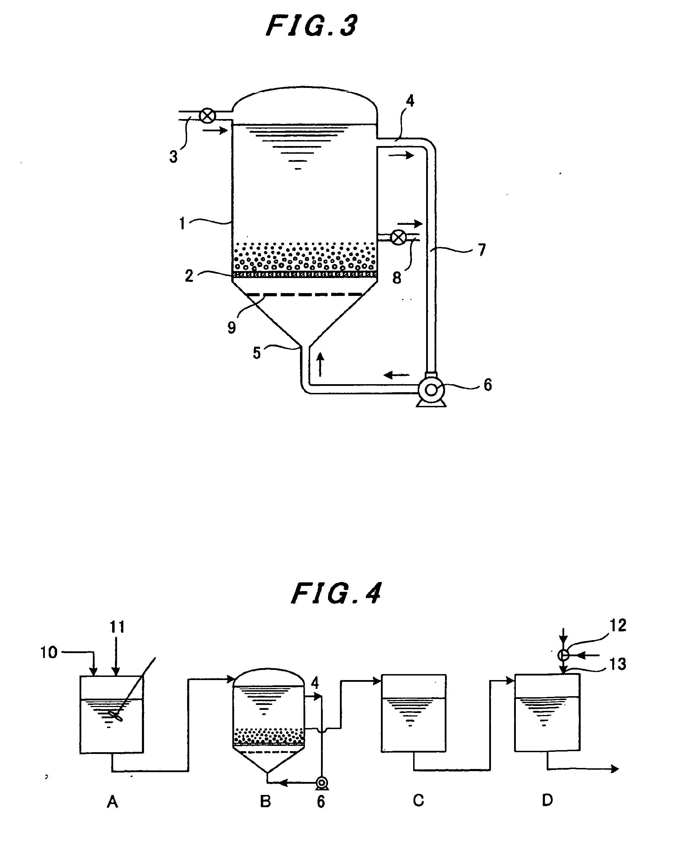

Fig. 3 is a brief cross-sectional view showing a preferable extraction apparatus used for the method of the present invention. A cylindrical container 1 is provided with a filter 2 of nonwoven fabric or filter cloth made of, for example, a fluorinated resin in the lower part thereof, an inlet port 3 for introducing an aqueous solution of an organic acid in the upper part thereof, an outlet port 4 for the solution in the upper part thereof, and a circulation inlet port 5 at the bottom thereof for circulation of the solution. Further, the container 1 is equipped with a pump 6 and a circulation pipeline 7 connecting the outlet port 4 and the circulation inlet port 5. The container 1 is also provided with an exit port 8 for discharging the solution and optionally with a rectifying plate 9 having a number of perforated holes for rectifying the flow of circulated solution in front of the filter. The diameter of the perforated holes opened for the plate 9 is larger in the peripheral portion while it is smaller in the central portion.

In practice of the method, a particulate natural radioactive material which has previously been divided to have a size of 20-150 mesh is placed on the filter 2 in such manner that an amount of the charged material corresponds to about 1/10 of the distance between the filter 2 and the outlet port 4, and then an aqueous solution of an organic acid such as citric acid and the like having a concentration of 0.05-10% by weight is introduced into the container 1 through the inlet port 3 in such manner that the liquid level of the solution is higher than the outlet port 4 for the solution.

The pump 6 is then started to effect circulation of the solution through the circulation pipeline 7 in the direction from the outlet port 4 to the circulation inlet port 5 whereby a fluidized bed of the natural radioactive material is formed on the filter 2 to attain extraction of radioactive decay products. The flowing velocity of the solution for circulation is so adjusted that the height of the fluidized bed of the natural radioactive material is 2-3 times as much as that of the material in stationary state.

This extraction treatment may be carried out at room temperature. Since the extraction treatment at such a lower temperature needs a long period of time until the solution reaches the desired concentration of the radioactive decay products, it is preferable in the present invention to use the organic acid solution heated at 50-100 °C. The use of the organic acid solution having such an elevated temperature affords an aqueous solution having a radiation dose of 0.68-13.7 Bq/g at an extraction time within 30 minutes to 10 hours.

The water having radioactivity thus obtained can be recovered from the exit port 8 of the container 1 and is stored, if necessary, in a storage tank (not shown).

The method of the present invention using the apparatus including the vessel 1 can be conducted batchwise as heretofore described or can be conducted as a continuous process by using a multistage sequence of the apparatus units with the discharge port of a stage connected to the inlet port of the next stage.

The present invention employing the fluidization-circulation type apparatus (contacting extraction tank) can produce radioactive aqueous solution in an extremely efficient way and the resultant water having radioactivity can be stored in a proper tank or can directly be supplied as "refined substitute for natural radioactive hot spas" to users, if necessary, after dilution and adjustment in radiation dose, temperature, and pH value of the aqueous solution.

In the following, the present invention will be explained in more detail by way of an example. It is to be construed, however, that the present invention is not limited by this example.

Incidentally, the following apparatuses or devices were used in the example.
(1) Magnetic separator:
   High gauss magnetic separator with a DC-rectifier, product name: Magnetic Separator Model ID, manufactured by TORSCO, average treatment capacity: 500-600 kg/hr; voltage scale: 0-10 A; magnetic scale: 0-50000 gauss; wiring voltage: 50 cycle, 200 V
(2) High tension AC electrostatic separator:
   product name: High Tension Separator Model HT20/210, manufactured by TORSCO, average treatment capacity: 850 kg/hr; DC 0-3A; memory index: 0-250 V; wiring voltage: 50 cycle, 200 V
(3) Classifier:
   Deck table made of aluminum, manufactured by TORSCO

### Example

500 Kilograms of heavy sand remained at the time of ore-dressing of cassiterite, i.e. tin ore, produced at a tin mine in Perak state, Malaysia was classified by water sieving by the aid of the above-mentioned classifier to obtain a fraction of ore which did not pass through 150 mesh screen while removing light sand (silica, feldspar, etc.) and then the collected ore was dried.

Using the above-mentioned magnetic separator, about 75% by volume of the ores was cut off under the magnetic condition of 8000 G (0.8 T) to remove iron-containing ores (magnetite, ilmenite, wolframite, ferrosilica sand, etc.).

The ore thus treated was then subjected to the treatment using the above-mentioned high tension AC electrostatic separator under the condition of 200 V and 3 A for about 1 hour whereby minerals other than monazite, xenotime, zircon, and quarts were removed. Using the magnetic separator again, the ore was treated under the magnetic condition of 25000 G (2.5 T) to 35000 G (3.5 T) for about 1 hour to separate monazite and xenotime.

In such a manner, a natural radioactive ore containing xenotime and monazite in a weight ratio of 1:10 was obtained. A result of a radioactive measurement of the product showed 227.8 Bq/g.

Extraction of the radioactive isotopes was then carried out by using an extracting tank made of stainless steel having the structure as shown in Fig. 3.

The tank was charged with the powdery natural radioactive ore up to a height of 10 cm from the filter 2 and filled with an aqueous solution of citric acid having a concentration of 0.5% by weight up to a height of 80 cm from the filter 2 introduced into the tank via the inlet port 3. The extraction treatment was then carried out by circulating the citric acid solution at 80 °C and at a flow rate of 50 ml/minute for 2 hours. Water having radioactivity of 8.2 Bq/g for use in hot bathing was thus obtained.

As a result of analysis of the water for use in bathing by way of γ-ray spectrometry showed existence of the nuclides such as ²²⁰Tn, ²¹²Pb, ²⁰⁸Tl, ²¹²Bli. No other radioactive nuclides could be detected, probably, due to the insufficient sensitivity of the instrument.

### Application Example

Using an apparatus system as shown in Fig. 4, an aqueous solution having radioactivity was prepared as follows:

In Fig. 4, A stands for a tank provided with a stirrer for preparing an acidic extraction liquid (i.e. for preparing an aqueous solution of citric acid by mixing water and citric acid), B stands for the fluidization-circulation type contacting extraction container (tank) which was a same type as that used in Example for contacting an aqueous solution of citric acid with a material containing radioactive isotopes in fluidized state to extract radioactive decay products, C stands for a storage tank for a concentrate (extracted aqueous solution), and D stands for an adjustment tank served for adjusting the concentrate via a 3-way valve by adding plain water and hot water to adjust the concentration of citric acid, pH value, and irradiation dose according to the condition for use as well as adjusting the liquid temperature, for example, within the range of 38-40°C. The aforementioned extraction tank B has a structure shown in Fig. 3 wherein a cylindrical container has a diameter of 100 cm and a height from the filter to the outlet port for the liquid is 100 cm. Used as the filter is a nonwoven fabric made of a fluorinated resin having a thickness of 1 mm.

In order to prepare water for bathing by the aid of the apparatus system shown in Fig. 4, a hot water kept at 100 °C was at the outset introduced into the tank A through a supply pipeline 10. Powdery citric acid was then added to the tank A through a supply pipeline 11 to prepare an aqueous solution of citric acid (pH: approximate 3) having a concentration of 1% by weight. The aqueous solution of citric acid thus prepared (1000 kg) was supplied to the extraction tank B charged with 50 kg (3 kg in terms of thorium) of the granular natural radioactive material obtained in Example. In this case, the liquid level was positioned above the outlet port 4. A pump 6 was driven to recycle the aqueous solution at such circulation velocity that the granular natural radioactive material formed a fluidized bed having a height of 2-3 times as much as the height thereof in stationary state. The extraction treatment was carried out for 4 hours at a temperature of 90-95 °C. In the course of the treatment, a solid mineral was not dissolved but radioactive decay products only were extracted.

The extraction liquid (concentrate) was then fed to the tank C for storage. A radioactivity of the resultant extraction liquid was 8.2 Bq/g. The extraction liquid was then fed to the adjustment tank D where the liquid was diluted with plain water and hot water in a mixing ratio of 1:1 introduced into the tank D via the 3-way valve 12 through the pipeline 13, whereby warm water kept at 38-40 °C having a radioactivity of 0.82 Bq/g was obtained. The concentration of citric acid in this warm water was 0.05% by weight while the pH value was 3.5. These values were almost equivalent to those of soft water of general drinkable weakly acidic hot spas.

As a result of γ-ray spectrometry, the warm water thus obtained showed existence of the nuclides of ²²⁰Tn, ²¹²Pb, ²⁰⁸Tl, and ²¹²Si. No other radioactive nuclides could be detected, probably, due to the insufficient sensitivity of the instrument. The radioactivity of the thoron-series nuclides in the warm water showed a half-life of about 12 hours.

According to the present invention, the method is advantageously carried out by employing the fluidization-circulation type contacting extraction container to produce physiologically useful hot or warm water having radioactivity. This produced water is free of any harmful radioactive material having a long half-life and can be produced in a large amount and can be diluted freely with water or hot water to make "substitute" for natural radioactive spas gushing out in only limited areas in the world. In addition, temperature, concentration, pH, and radioactivity can freely be adjusted so that people can repeatedly take bath with the product of the invention without any fear of radioactivity.

## Claims

1. A method for the preparation of water having radioactivity for use in bathing enriched in the content of thoron-series isotopes which comprises the steps of (A) subjecting a natural radioactive ore containing both of thoron-series isotopes and radon-series isotopes to a separation treatment for selectively eliminating the radon-series isotopes only thereby decreasing the content of the radon-series isotopes and (B) bringing the natural radioactive ore thus treated into contact with an aqueous solution of an organic acid to extract radioactive decay products in the natural radioactive ore with the aqueous solution.

2. The method according to claim 1, wherein the natural radioactive ore used in the step (A) is a heavy sand collected from a pegmatite-based sand deposit and the step (A) is conducted by separation of thorium-series monazite from uranium-series xenotime through mineral dressing in such a manner that the amount of the xenotime is reduced to decrease the content of the radon-series isotopes.

3. The method according to claim 1, wherein the concentration of the aqueous solution used in the step (B) is in the range from 0.01 to 50% by weight.

4. The method according to claim 1, wherein the organic acid is citric acid.

5. A method for the preparation of water having radioactivity for use in bathing enriched in the content of thoron-series isotopes by the aid of a fluidization-circulation-type contacting extractor having a cylindrical container provided with a filter in the lower part thereof, an outlet port for a liquid in the upper part thereof, a circulation inlet port for a liquid at the bottom thereof, and a circulation pipeline connecting the outlet port and the circulation inlet port via a pump, which method comprises the steps of (a) putting a particulate material containing radioactive isotopes on the filter, (b) introducing an aqueous solution of an organic acid into the container so that the liquid level of the solution is higher than the outlet port thereof for the liquid, and (c) circulating the aqueous solution by way of the pump and the circulation pipeline so as to form a fluidized bed of the particulate material containing radioactive isotopes, wherein a natural radioactive ore containing thoron-series isotopes and radon-series isotopes is subjected to a separation treatment for selectively eliminating the radon-series isotopes only thereby decreasing the content of the radon-series isotopes and the resultant product is employed as the aforementioned particulate material containing radioactive isotopes.

## Patentansprüche

1. Verfahren zur Aufbereitung von Radioaktivität enthaltendem Badewasser, das mit Isotopen aus der Thorium-Reihe angereichert ist, mit den Schritten: (A) Unterziehen eines natürlichen radioaktiven Erzes, welches sowohl Isotope aus der Thorium-Reihe als auch Isotope aus der Radon-Reihe enthält, einer Trennbehandlung, um selektiv die Isotope aus der Radon-Reihe zu eliminieren, wobei nur **dadurch** der Gehalt an Isotopen der Radon-Serie verringert wird und (B) Inkontaktbringen des natürlichen radioaktiven Erzes mit einer wässerigen Lösung einer organischen Säure, um radioaktive Zerfallsprodukte aus dem natürlichen radioaktiven Erz mit der wässerigen Lösung zu extrahieren.

2. Verfahren nach Anspruch 1, wobei das natürliche radioaktive Erz, welches im Schritt (A) verwendet wird, ein schwerer Sand ist, der von einer pegmatithaltigen Sandablagerung gesammelt wird, und Schritt (A) durchgeführt wird, indem Monazit aus der Thorium-Serie von Xenotim aus der Uran-Serie durch Mineralveredelung derart getrennt wird, dass die Menge an Xenotim reduziert wird, um den Gehalt an Isotopen aus der Radon-Serie zu vermindern.

3. Verfahren nach Anspruch 1, wobei die Konzentration der im Schritt (B) verwendeten wässerigen Lösung zwischen 0,01 bis 50 Gew.-% liegt.

4. Verfahren nach Anspruch 1, wobei die organische Säure Zitronensäure ist.

5. Verfahren zur Aufbereitung von Radioaktivität enthaltendem Badewasser, welches mit Isotopen aus der Thorium-Reihe angereichtert ist, mittels eines Verflüssigungs-Zirkulationstyp-Kontaktextraktors mit einem zylindrischen Behälter, der in seinem unteren Bereich mit einem Filter versehen ist, einem Flüssigkeitsauslass in dessen oberem Bereich, einem Zirkuationseinlass für eine Flüssigkeit in dessen Bodenbereich und mit einer Zirkulationsleitung, die den Auslass und den Zirkulationseinlass über eine Pumpe verbindet, wobei das Verfahren folgende Schritte aufweist: (a) Auflegen von staubförmigem Material mit radioaktiven Isotopen auf den Filter, (b) Einführen einer wässerigen Lösung einer organischen Säure in den Behälter, sodass der Flüssigkeitsspiegel der Lösung höher liegt als der Auslassanschluss für die Flüssigkeit, und (c) Zirkulieren der wässerigen Lösung mit Hilfe der Pumpe und der Zirkulationsleitung, um ein verflüssigtes Bett aus dem zerstäubten Material zu bilden, welches radioaktive Isotope enthält, wobei das radioaktive natürliche Erz mit Isotopen aus der Thorium-Reihe und mit Isotopen aus der Radon-Reihe einer Trennbehandlung unterzogen wird, um selektiv die Isotope aus der Radon-Reihe zu eliminieren, wobei nur **dadurch** der Gehalt an Isotopen aus der Radon-Reihe verringert wird, und das erhaltene Produkt wird als das oben genannte zerstäubte Material mit radioaktiven Isotopen verwendet.

## Revendications

1. Méthode pour la préparation d'une eau présentant une radioactivité pour une utilisation dans un bain enrichi en teneur en isotopes de la série toron qui comprend les étapes de (A) soumettre un minerai radioactif naturel contenant à la fois des isotopes de la série toron et des isotopes de la série radon à un traitement de séparation pour éliminer sélectivement les isotopes de la série radon uniquement pour ainsi diminuer la teneur en isotopes de la série-radon et (B) mettre le minerai radioactif naturel ainsi traité en contact avec une solution aqueuse d'un acide organique pour extraire les produits radioactifs de dégradation dans le minerai radioactif naturel par la solution aqueuse.

2. Méthode selon la revendication 1, où le minerai radioactif naturel utilisé à l'étape (A) est un sable lourd collecté à partir d'un dépôt de sable à base de pegmatite et l'étape (A) est entreprise par séparation de la monazite de la série thorium de la xénotine de la série uranium par triage minéral de manière que la quantité de xénotine soit réduite pour diminuer la teneur en isotopes de la série radon.

3. Méthode selon la revendication 1, où la concentration de la solution aqueuse utilisée à l'étape (B) est comprise entre 0,01 et 50% en poids.

4. Méthode selon la revendication 1, où l'acide organique est de l'acide citrique.

5. Méthode pour la préparation d'une eau présentant une radioactivité pour une utilisation dans un bain enrichi en teneur en isotopes de la série thoron par l'aide d'un extracteur à contact du type à fluidification-circulation ayant un conteneur cylindrique pourvu d'un filtre à sa partie inférieure, d'un orifice de sortie pour un liquide à sa partie supérieure, d'un orifice d'entrée de circulation pour un liquide à sa partie inférieure, et d'un pipeline de circulation connectant l'orifice de sortie et l'orifice d'entrée de circulation via une pompe, laquelle méthode comprend les étapes de (a) mettre une matière particulaire contenant des isotopes radioactifs sur le filtre, (b) introduire une solution aqueuse d'un acide organique dans le conteneur de façon que le niveau du liquide de la solution soit plus élevé que son orifice de sortie pour le liquide, et (c) mettre en circulation la solution aqueuse par le moyen de la pompe et du pipeline de circulation afin de former un lit fluidifié de la matière particulaire contenant des isotopes radioactifs, où un minerai radioactif naturel contenant des isotopes de la série toron et des isotopes de la série radon est soumis à un traitement de séparation pour l'élimination sélective des isotopes de la série radon uniquement pour ainsi diminuer la teneur en isotopes de la série radon et le produit résultant est employé en tant que matière particulaire ci-dessus mentionnée contenant des isotopes radioactifs.
